# EUROPEAN PATENT APPLICATION

(11) **EP 2 835 365 A1**
(43) Date of publication of application: **11.02.2015**
(21) Application number: 13306126.7
(22) Date of filing: 06.08.2013
(51) Int. Cl.: C07C 231/08, C07D 207/267, C07D 207/408

(54) **A process to produce N-ethenyl-N-alkyl-alkylamides**

(71) Applicant: S.P.C.M. SA, 42160 Andrézieux Bouthéon (FR)
(72) Inventor: Favero, Cédrick, 42610 Saint Romain le Puy (FR); Kieffer, Johann, 42000 Saint Etienne (FR)
(74) Representative: Cabinet Laurent & Charras

(57) **Abstract**

A process to produce N-ethenyl-N-alkyl-alkylamide including the steps of reacting at least a N-monoalkyl-alkylamide with a reactant including 1,3-dioxolan-2-one in the presence of at least a catalyst to form an intermediate, and of decarboxylating said intermediate to synthesize N-ethenyl-N-alkyl-alkylamide.

## Description

The present invention relates to a process to produce of N-ethenyl N-alkyl alkyl amide, and particularly, to the synthesis of N-ethenyl N-alkyl alkyl amide using 1,3-dioxolan-2-one and N-alkyl alkyl amide or N-alkyl lactam as starting materials.

N-ethenyl N-alkyl alkyl amide are a class of reactive chemical with potentially useful properties in that it free-radically polymerizes to produce water-soluble poly (N-ethenyl N-alkyl alkyl amide) and also undergoes controlled radical polymerization using RAFT methodology. Badesso, R.J. ; Nordquist, A. F.; Pinschmidt, Jr. R. K.; and Sagl, D. J. "Hydrophilic polymers : performance with Environmental Acceptance", Glass, E.; Ed.; America Chemical Society, Washington, DC, 1995, p489. These polymers are used in applications such as cosmetics, EOR, drilling, detergency, agriculture, construction, paper, mining reagents etc...

All the reaction routes to N-ethenyl N-alkyl alkyl amides are well documented in a book "water soluble poly-N- vinylamides", synthesis and physicochemical properties, Yury E. Kirsh, Wiley, 1998, and in water soluble monomers, Kobunshi Kato, 1989, 38(10), pp 507-17 from Masaya Kameya and Yoshida Kensei (Nitto Kako Co., Ltd.). Only a few are known commercial routes to N-ethenyl N-alkyl alkyl amide.

One of the most significant route is based on alpha elimination of leaving groups in alpha-substituted N- ethyl N-alkyl alkyl amide. Depending on companies, the alpha substituent can be butyrolactam in ethylidene n,n'-bis-butyrolactam (GAF- USA in WO 1991002720) A1), methanamide (Air Product Chemicals - USA), cyanohydric acid (BASF- Germany), Methanol (Mitsubishi Japan), etc. More details on the reaction conditions, yields and drawbacks can be found in Journal für praktische Chemie, Volume 342, Issue 2, pages 115-131, February 2000, N-Vinylformamide - Syntheses and Chemistry of a Multifunctional Monomer, Michael Kröner Dr., Jacques Dupuis Dr.,Manfred Winter1, 2000 WILEY-VCH Verlag GmbH, Weinheim, Fed. Rep. of Germany. More recently, the inventor in WO 2004/020395 has studied a new route for the production of N-ethenyl N-methanamide including the steps of reacting hydroxyl ethyl methanamide with a reactant including at least one cyclic anhydride group to form an ester, and dissociating (or cracking) the ester to synthesize N-ethenyl N-methanamide and a compound including at least one diacid group. This process has never been up scaled due to the formation of large amounts of acid waste. All the production routes through the alpha substitution suffer from high temperature cracking, handling of difficult chemicals, produce unstable monomer, require expensive multi step process and purification with a lot of downtime, and require large amount of polymerization inhibitors to ensure an adequate in -process and storage stability for the resulting monomer. The monomer thus produced has bad polymerization properties, is very expensive and is not stable.

Another significant commercial route is the Reppe route involving acetylene on N-ethyl N-alkyl alkyl amide. The Reppe reaction (Reppe W. (1949) acetylene chemistry. Ch A Meyer, New York, p68) is commercially in use for the production of N-ethenyl caprolactam. The reaction is well documented in a paper from Kononov, N.F. et Al. (1977) "New technology of some syntheses on the basis of acetylene". Nauka, Moscow, p. 174. The Reppe reaction has also been applied successfully for the production of N-ethenyl N-butyrolactam, also called N-vinyl pyrrolidone by company GAF, then International Specialty Products, now Ashland - USA (see Process for the preparation of N-vinyl lactams US 4873336 A) and by BASF, Germany (US 2011/0034706 A1).

This production route is not very advantageous because the investment for the production plant is high, due to safety apparatus to be implemented on such plant handling acetylene under pressure along with the risk of possible explosion. In addition, the desired yield cannot be high enough unless the reaction is conducted under very severe reaction conditions.

Moreover the harsh conditions used in such process lead to formation of heavy by-products decreasing the overall quality and polymerization capabilities of the final N-ethenyl N alkyl alkyl amide. For instance, in the production of N-ethenyl butyrolactam, these impurities are butyrolactam adducts such as but not limited to N-(4-butyramido)-2-pyrrolidone , N-(4-butyronitrile)-2-pyrrolidone, 4-(2-pyrrolidonyl)-butyric acid, 1,4bis (2-oxo-1-pyrrolidonyl)-1-butanone and 1-(1-pyrrolin-2-yl)-2-pyrrolidone. The last impurity mentioned is problematic in current process as it is well-known accelerator for Nylon-4 manufacturing. This lead in the current process to polymerization deposit, equipments and catalyst fouling and so high maintenance cost due to down time for cleaning.

A last commercial route is through beta-substituted N-alkyl alkyl amide elimination reaction involving dehydration of N-hydroxyethyl N-alkyl alkyl amide. This vapor phase dehydratation process is commercialy in use for the production of N-ethenyl butyrolactam. The reaction is well documented in a paper from Yuuji Shimasaki and Al (2010) "Development of a new production process for N-vinyl-2-Pyrrolidone" Catal Surv Asia Vol 14 p.132-139).

The dehydration process has also been applied successfully by BASF company in US 5,994,562 using aluminium based zeolite and by Pine-Sci Kuo and Al in US 5,569,770 using N-beta hydroxyethyl N-butyrolactam as reactant in presence of mixed oxide of group IV element such as zirconium, titanium, tin, silicon and hafnium oxide.

This production route suffers of several drawbacks such as high cracking temperature, expensive use of catalyst and catalyst prone to fouling with time. These issues lead to multiple downtimes to regenerate the catalyst, intensive cleaning of equipments due to the formation of polymers into the process equipments. To avoid downtime, a high amount of polymerization inhibitor is used which in turns leads to bad monomer quality.

Manufacturers of those N ethenyl N alkyl alkyl amide, synthetized by beta substituted N-alkyl alkyl amide elimination reaction, are aware of this drawback and beyond the production of monomers they have applied for patents on stabilization and purification to solve partially these problems. This is well described in the patent JP 2007/238471A whereas a cumbersome invention is provided to keep the quality of N-ethenyl N alkyl alkyl amide in convenient quality for further polymerization by pressurizing with nitrogen every storage equipment such as reactor, separation column, heat exchanger,storage tank, process equipment, drum, totes, buffer tank...etc. For instance the N-ethenyl N alkyl alkyl amide produced is further purified by melt recrystallization such as described in the patent US WO2007/094467 A1 from Nippon Shukobai company. The same is increasing the capital expenditure of the manufacturing plant, decreasing the overall yield, and increase the energy consumption, the overall benefit is not economical and practical.

In a similar matter, beta substituted hydroxyethyl butyrolactam derivative reacted with maleic anhydride has been studied with similar issues during gas phase cracking. A poor monomer quality is related.

Another route quite intensively studied, but only at small scale to our knowledge is through the reaction between N-alkyl alkyl amide and vinyl ether under pressure; such as with ethenyl methanoate. A review paper is available in Organic letters, 2004, vol.6, N°.11 1845- 1848 - formation of enamides via palladium (II) catalyzed vinyl transfer from vinyl ethers to nitrogens nucleophiles from university of Wisconsin (Brice, Meerdink and Stahl).

Such route involves the use of hazardous reactants such as vinyl alkyl ether and more particularly the use of expensive and not available catalysts based on palladium moieties, with additional drawback on recycling such catalysts on industrial scale.

It is desirable to develop alternative routes to the synthesis of N- ethenyl N-alkyl alkyl amide without disadvantages of the prior art.

### Summary of the invention

The present invention is about a process to produce N-ethenyl N-alkyl alkyl amide including the steps of reacting 1,3-dioxolan-2-one as the raw material with a reactant including at least a N-mono alkyl alkyl amide, and decarboxylating the formed intermediate to synthesize N-ethenyl N-alkyl alkyl amide using a compound including at least a catalyst. The intermediate can be dissociated using heat in addition to a catalyst.

The said synthesis method has the advantages of producing high purity N-ethenyl N alkyl alkyl amide derivatives for use in polymerization with excellent shelf life with minimum polymerization inhibitor due to mild production conditions.

The mild production conditions also have the advantage of avoiding self-polymerization in the production process and purification sections and allows low maintenance and down time on production unit.

### Details of the invention:

The object of the invention is attained by using 1,3-dioxolan-2-one as a reactant along with N-mono alkyl alkyl amide in presence of a catalyst. In a preferred embodiment, the said catalyst is an inorganic solid alkali or a nitrogen-containing compound with weak nucleophilicity.

Therefore, the invention concerns a process to produce N ethenyl N alkyl alkyl amide including the step of reacting at least N-mono alkyl alkyl amide with a reactant including 1,3-dioxolan-2-one in the presence of at least a catalyst to form an intermediate, and decarboxylating said intermediate to synthesize N- ethenyl N-alkyl alkyl amide.

The 1,3-dioxolan-2-one, from the carbonic ester family, is preferentialy used as reactant for the invention. Possible reaction with such carbonic ester is the decarboxylation to form ethenyl moities as described in the Ulmann's (p442 vol 6, section pyrolysis). In the present invention, 1,3-dioxolan-2-one is used to form ethenyl bond. According to the state of the art, such carbonic ester can only be cracked at temperature above 300°C.

N-alkyl alkyl amide suitable for the present invention has the following formula I:

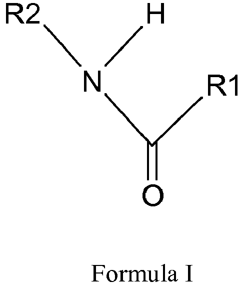

Wherein when R1 and R2 are linear alkyl chains :
R1 = H or CH₃ or a linear or branched alkyl containing not more than 6 carbons and optionally comprises O, N, S and/or P atoms,
R2= H or CH₃ or a linear or branched alkyl containing not more than 6 carbons and optionally comprises O, N, S and/or P atoms,
R1 + R2 ≤ 6 carbons

Wherein when R1 and R2 form a cycle :
R1+R2= an alkyl chain of at least 3 carbons to not more than 6 carbons and optionally comprises O, N, S and/or P atoms.

N-mono alkyl alkyl amides where R1 and R2 have linear alkyl chains suitable for the present invention are preferably chosen from the group comprising methanamide, methyl methanamide, methylacetamide and acetamide. More preferably the N-alkyl alkyl amide is methanamide.

N-mono alkyl alkyl amides where R1 and R2 form a cycle suitable for the present invention are preferably chosen from the group comprising caprolactam, valerolactam, butyrolactam and succinimide. More preferably the N-alkyl alkyl amide is butyrolactam.

Preferably, N-mono alkyl alkyl amide are chosen from the group comprising butyrolactam, methylacetamide, methanamide, succinimide, caprolactam, acetamide.

In a preferred embodiment, the N-alkyl alkyl amide has a structure increasing its nucleophilicity such as the base catalyst reacts preferentially with the labile hydrogen of the amide group instead of creating a competing reaction with the carbonate ester.

The N-alkyl alkyl amide of increased nucleophilicity are amides with a cyclic structure or with higher alkyl chains length on R2 since it favors the electron donating effect in order to push the reaction toward amide / 1,3-dioxolan-2-one reaction to form N-ethenyl, N- alkyl alkyl amide. In a further preferred embodiment, cyclic N-alkyl alkyl amide, or N-lactam are preferred since they have not only an increased nucleophilicity but also are sterically hindered. Therefore, they preferably react with the methylene carbon of 1,3-dioxolan-2-one to form a beta carbonate of N-ethyl N-alkyl lactam, instead of reacting with the carbonyl function of the 1,3-dioxolan-2-one, which is an undesired side reaction.

In a preferred aspect of the invention, in the formula I, where R1 and R2 are linear alkyl chains, R2 is not H atom.

In a preferred aspect of the invention, in the formula I, where R1 and R2 are linear alkyl chains, R1 and R2 are not H atoms.

In the reaction of cyclic carbonates with regular alkaline reagents, quite often the alkaline nucleophilic molecules/ions attack the carbonyl carbon atom of carbonates; for instance, under the effect of catalyst, the aliphatic amine with strong nucleophilicity attacks the carbonyl carbon atom of carbonates, the ring is opened at the end, urethane structure of hydroxyl is formed.

Without being bound to any theory, it is supposed that the nitrogen group in the amide has some basic character and in presence of another basic material, an equilibrium can be formed between the two chemicals. A catalytic amount of negatively charged amide is formed. This non isolated intermediate can react with the 1,3-dioxolan-2-one, on the methylene carbon to open the ring structure. A beta substituted N-ethyl, N-alkyl alkyl amide (supposed intermediate) is obtained and is then decarboxylated for the production of the targeted N-ethenyl, N-alkyl alkyl amide.

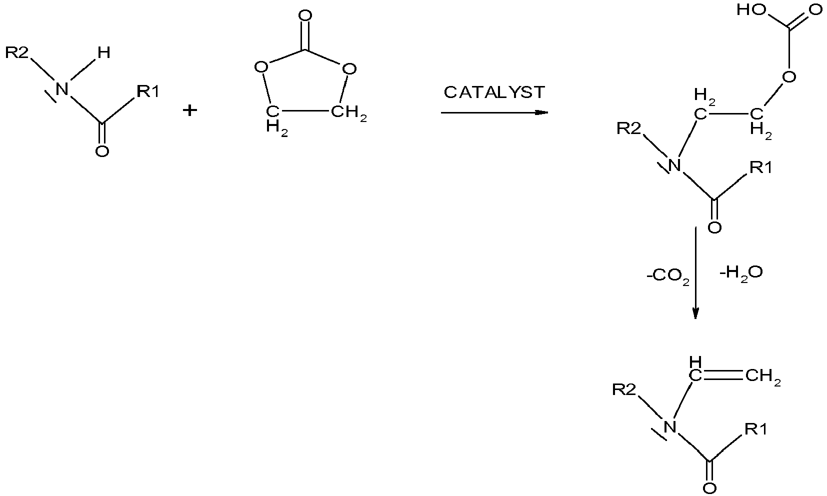

The mass ratio 1,3-dioxolan-2-one: N-alkyl alkyl amide is comprised between 1:99 to 99:1, preferentially between 1:10 to 10:1.

Compounds can be added in any order. For example 1,3-dioxolan-2-one can be poured on N-alkyl alkyl amide or vice versa.

In an alternate mode, the reaction can be conducted under pressure. The reaction can be conducted in a batch or a serial of batch vessels or in continuous process, for instance when a fixed catalyst bed is employed.

Any catalyst useful to perform the reaction is considered in the scope of the present invention. Since the competition reaction must be avoided, the basic catalyst is preferred. According to a first embodiment of the invention, the catalyst is an inorganic solid alkali. They can be used as homogeneous (soluble in reaction media) or heterogeneous catalysts (solid, ion exchange resin, catalyst bed or packing). Inorganic solid alkali is preferably chosen from the group comprising oxides, hydroxides, carbonates and bicarbonates of an alkali metal from the group I A or II A or of ammonium, such as sodium carbonate, or solid material such as active alumina, cerium oxide, zinc oxide, chromium oxide, zirconium oxide, thorium oxide, lanthanium oxide and neodymium oxide.

In a preferred embodiment, the catalyst is an organic base such as organic amidine, guanidine and phosphazene type bases. They are typical strong nonionic bases and show good solubility in most organic solvents, even at low temperature.

An example of typical suitable catalyst is shown herebelow :

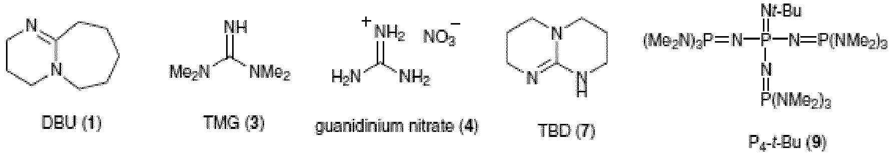

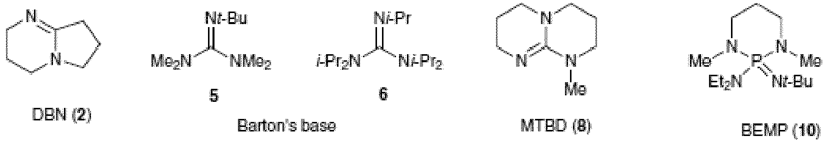

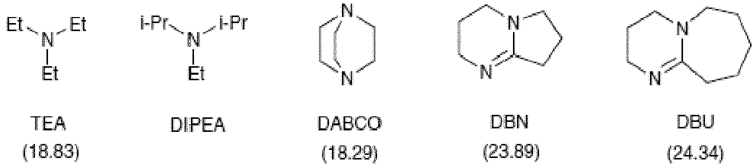

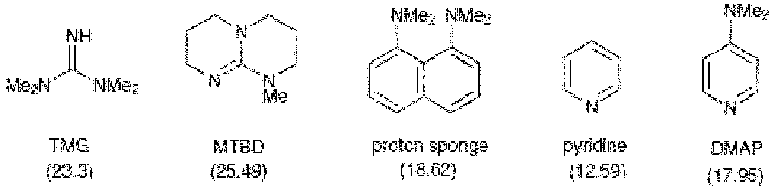

Preferentially the catalyst is chosen from the group comprising 1, 8-Diazabicyclo [5,4,0] undec-7-ene (DBU), 1, 5-Diazabicyclo [4.3.0] non-5-ene (DBN), 1, 5, 7-Triazabicyclo [4.4.0] dec-5-ene (TBD), 7-methyl-1, 5, 7-Triazabicyclo [4.4.0] dec-5-ene (MTBD), 1, 1, 3, 3-tetramethylguanidine (TMG) or potassium bis(trimethylsilyl) amide (KHMDS). More preferably the catalyst is chosen between 1,5-diazabicyclo[4.3.0]non-5-ene (DBN) and 1,8-diazabicyclo[5.4.0]undec-7-ene (DBU).

The catalyst dosage is comprised between 0,1 to 10%, preferably between 0,5 to 5% of the total weight of the entire reaction system. A too low quantity of catalyst will not generate enough conversion and selectivity. A too high quantity will increase the cost and lead to difficulties during purification.

The process optionally further includes the presence of a solvent known by the skilled man of the art. It includes but not limited to ethyl acetate, MIBK, N-methyl-pyrrolidone, acetone, tetrahydrofuran, dimethylformamide, dimethylacetamide, DMSO, sulfolane, toluene, xylene, benzene, cresol. The solvent can be as well an ionic liquid or in supercritical CO2 well known by the skilled man of the art.

In a preferred embodiment, the N-alkyl alkyl amide or the 1,3-dioxolan-2-one is used as the solvent of the reaction.

Preferably, the mass percent of solvent in the reaction mixture is less than 80%, preferentially less than 50 and more preferentially less than 25% (neat reaction).

In the present invention preferentially the intermediate is decarboxylated at temperature from 20 to 150°C, preferentially from 30 to 120°C, and more preferentially from 40 to 100°C.

A lower temperature improves the process by preserving the N-ethenyl N-alkyl alkyl amide of self polymerization during the manufacturing process and improving overall product quality such as shelf life, color, reactivity, etc...

According to another aspect of the present invention, the crude N ethenyl N alkyl alkyl amide can be purified by any know state of the art process. We can mention for example by distillation as described in the US patent US 6436243 and US 7037412 or by melt crystallization as described in US 7138528, US 20090088579, US 6703511 and US 5329021.

According to another aspect of the present invention, the N-ethenyl N alkyl alkyl amide is obtained in a liquid or molten solid form, having a purity >95%, preferentially >98%, preferentially >99,9% and more preferentially >99,99%.

In a specific aspect of the invention, the process comprises further adding stabilizers to the N-ethenyl N alkyl alkyl amide before, during or after production. We can mention for example compounds belonging to the amine group of chemicals as N,N'-di-sec.-butyl-p-phenylendiamine (Kerobit BPD), 4,4'-Bis (α,α,dimethylbenzyl) diphenylamine, 4 isopropylamino diphenylamine, p-amino diphenylamine, N-(1,3-dimethylbutyl)-N'phenyl-p-phenylenediamine, N,N'-di-dec-butyl-p-phenylenediamine, mixtures of substituted p-diphenylamines, mixtures of octylated diphenylamines, ammonium N-nitrosophenylhydroxylamine, aluminium N-nitrosophenylhydroxylamine; salt of EDTA as disodium, diammonium or tetraammonium; phenolic free radical chemicals such as, HQ (hydroquinone), MEHQ (methyl- hydroquinone), BHT (butylated hydroxytoluene) and BHA (butylated hydroxyanisole) ; or 2,6-di-tert.-butyl-phenol (Kerobit TP 26), methoxyphenol, phenothiazine, 2, 6-di-tert-butyl-p-cresol or copper acetate, caustic soda or hydroxyl tetramethyl piperidinyl oxy (HTPO).

Since water is generated in the reaction system during reaction, in order to boost the reaction process and reduce the number of side reactions, the water formed during the reaction can be removed during or after the reaction. The water can be removed for instance by use of a dehydrating agent or by distillation. In case a distillation is performed, vaccum can be applied to the reaction mixture. If appropriate a solvent can be used to lower the temperature to ease the water removal.

Another aspect of the invention concerns the polymer made of the N ethenyl N alkyl alkyl amide obtained by the process described before.

Therefore, the invention also concerns a process for manufacturing N ethenyl N alkyl alkyl amide based polymer comprising polymerizing N alkyl alkyl amide monomers obtained according to the process above explained.

In a specific embodiment, the N ethenyl N alkyl alkyl amide obtained by the process described before is copolymerized with at least one comonomer. Comonomers can be non-ionic, anionic, cationic or zwitterionic.

The anionic monomers which may be used in the context of the invention may be chosen in particular from monomers presenting acrylic, vinyl, maleic, fumaric or allylic functionalities and may contain a carboxylate, phosphonate, phosphate, sulfate or sulfonate group or another anionically charged group. The monomer may be acidic or may be in the form of a salt or of the corresponding alkaline-earth metal or alkali metal of such a monomer. Preferred monomers belonging to this class are, for example, acrylic acid, methacrylic acid, itaconic acid, crotonic acid, maleic acid, fumaric acid and monomers of strong acid type bearing, for example, a function of sulfonic acid or phosphonic acid type such as 2-acrylamido-2-methylpropanesulfonic acid, vinylsulfonic acid, vinylphosphonic acid, allylsulfonic acid, allylphosphonic acid, styrenesulfonic acid and the water-soluble alkali metal, alkaline-earth metal and ammonium salts thereof.

The nonionic monomer(s) which may be used in the context of the invention may be chosen in particular from the group comprising water-soluble vinyl monomers. Preferred monomers belonging to this class are, for example, acrylamide, methacrylamide, N-isopropylacrylamide, N-diethylacrylamide, N,N-dimethylacrylamide and N-methylolacrylamide. Also, use may be made of N-vinylformamide, N-vinylacetamide, N-vinylpyridine and N-vinylpyrrolidone, acryloylmorpholine (ACMO) and diacetone acrylamide.

The cationic monomer(s) which may be used in the context of the invention may be chosen in particular from monomers of the acrylamide, acrylic, vinyl, allyl or maleic type having a quaternary ammonium functional group. Mention may be made, in particular and without limitation, of quaternized dimethylaminoethyl acrylate (ADAME), quaternized dimethylaminoethyl methacrylate (MADAME), dimethyldiallylammonium chloride (DADMAC), acrylamidopropyltrimethylammonium chloride (APTAC) and methacrylamidopropyltrimethylammonium chloride (MAPTAC).

The polymer can be structured and can have the form of a star, a comb, or has pending groups of pending chains on the side of the main chain, or can be branched or even crosslinked.

In order to form a structured polymer, the polymerization of the monomers is generally conducted in the presence of at least one structuring agent which may be chosen from the group comprising polyethylenically unsaturated monomers (having at least two unsaturated functional groups), such as for example vinyl, allyl, acrylic and epoxy functional groups and mention may be made for example of methylene bisacrylamide (MBA), triallyamine, polyethylene glycol diacrylate, or alternatively using macro initiators such as polyperoxides, polyazo compounds and polytransfer agents such as polymercaptan polymers.

Others structuring agents are oligomeric or polymeric functional chemicals such as polyethyleneimine (PEI), polyvinylalcohol (PVOH) or hofmann degradation polymer, which can be added before or during polymerization at level up to 15% in polymerization mixture.

The polymerization of the monomers can optionally be conducted in the presence of at least one transfer agent which limits the length of the polymeric chains which may be chosen from the group comprising sulfurous acid (salts), hydrogen sulfite (salts), pyrosulfurous acid (salts), phosphorous acid (salts), phosphinic acid (salts), thioglycolic acid, octyl thioglycolate, thiopropionate, octyl thiopropionate; mercapto group-containing compounds such as n-dodecyl mercaptan, t-dodecyl mercaptan, ethylene glycol dithioglycolate, ethylene glycol dithiopropionate, 1,4-butanediol thioglycolate, trimethylol propane trithioglycolate, trimethylol propane trithiopropionate, pentaerythritol tetrakisthioglycolate, pentaerythritol tetrakisthiopropionate, dipentaerythritol hexakisthioglycolate, and dipentaerythritol hexakisthiopropionate; mercaptan compounds such as butanethiol, octanethiol, decanethiol, hexadecane thiol, octadecane thiol, cyclohexylmercaptan, thiophenol, mercaptoethanol, thioglycerol, thiomalic acid, and 2-mercaptoethanesulfonic acid; and secondary alcohols such as isopropanol, and glycerin, and amino alkyl mercaptan compound such as aminoethan thiol, aminopropane thiol and aminobutane thiol.

The water-soluble polymers using the N ethenyl N alkyl alkyl amide of in the invention do not require the development of a particular polymerization process. They may be obtained via any polymerization technique that is well known to those skilled in the art (solution polymerization, suspension polymerization, gel polymerization, precipitation polymerization, emulsion (aqueous or inverse) polymerization, optionally followed by a step of spray-drying, suspension polymerization, inverse suspension polymerization, micellar polymerization, optionally followed by a step of precipitation, post-hydrolysis or co-hydrolysis polymerization, radical "templates" polymerization or controlled radical polymerization. The water-soluble polymers can be obtained as an emulsion (inverse), a powder or any others liquid or solid forms.

The obtained polymer can be modified by different known reactions. We can cite for example the basic or acidic hydrolysis of the amide groups or the hofmann degradation or post-grafting of functional molecules on polymer using for example carbodiimide as coupling reagent.

Another aspect of the invention is the use of said polymer including the N ethenyl N alkyl alkyl amide obtained by the process described before in water treatment, oil & gas recovery, paper, cosmetic, detergency, pharmaceutical, mining reagents, agriculture, drilling, construction... etc

### Examples

To a 1 liter four necked flask with thermometer, dessicant tube, electrical stirrer, Dean Stark apparatus, is added 85,1g (1 mol) of butyrolactam, 130g (1.1 mol) of 1,3-dioxolan-2-one, 500ml of xylene , 300mg of 4-hydroxy-TEMPO as inhibitor and 6,4g of catalyst 1, 8-Diazabicyclo [5,4,0] undec-7-ene (DBU).

The whole reaction mass is heated at 100°C to react during 30mn. Once the reaction of decarboxylation start, water is gradually generated and separated along with the water carrying agent. The reaction stops after temperature reach 120°C after 3h and no longer water is generated.

The resulting product is further separated and the fraction at 90°C /9mmHg is collected. The product yield 82,13% of N-ethenyl butyrolactam with a purity of 99,3%.

## Claims

1. A process to produce N ethenyl N alkyl alkyl amide including the step of reacting at least N-mono alkyl alkyl amide with a reactant including 1,3-dioxolan-2-one in the presence of at least a catalyst to form an intermediate, and decarboxylating said intermediate to synthesize N- ethenyl N-alkyl alkyl amide.

2. A process according to the claim 1 wherein N-alkyl alkyl amide has the following formula I: 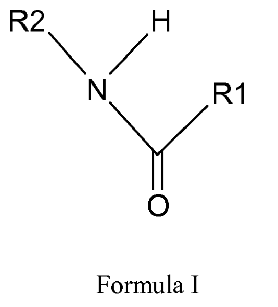 Wherein when R1 and R2 are linear alkyl chains :
R1= H or CH₃ or a linear or branched alkyl containing not more than 6 carbons and optionally comprises O, N, S and/or P atoms.
R2= H or CH₃ or a linear or branched alkyl containing not more than 6 carbons and optionally comprises O, N, S and/or P atoms.
R1 + R2 =< 6 carbons
Wherein when R1 and R2 form a cycle :
R1+R2= an alkyl chain of at least 3 carbons to not more than 6 carbons and optionally comprises O, N, S and/or P atoms.

3. A process according to the claim 1 wherein N-mono alkyl alkyl amides where R1 and R2 have linear alkyl chains are chosen from the group comprising methanamide, methyl methanamide, methylacetamide and acetamide.

4. A process according to the claim 1 wherein N-mono alkyl alkyl amides where R1 and R2 form a cycle are chosen from the group comprising caprolactam, valerolactam, butyrolactam and succinimide.

5. A process according to the claim 1 wherein N-mono alkyl alkyl amide are chosen from the group comprising butyrolactam, methylacetamide, methanamide, succinimide, caprolactam, acetamide.

6. A process according to any of claim 1 to 5 wherein the catalyst is an inorganic solid alkali preferably chosen from the group comprising oxides, advantageously cerium oxide, zinc oxide, chromium oxide, zirconium oxide, thorium oxide, lanthanium oxide and neodymium oxide, hydroxides, carbonates and bicarbonates of an alkali metal from the group I A or II A or of ammonium and active alumina.

7. A process according to any of claim 1 to 5 wherein the catalyst is an organic base chosen from the group comprising organic amidine, guanidine and phosphazene type bases.

8. A process according to any of claim 1 to 5 wherein the catalyst is chosen from the group comprising:

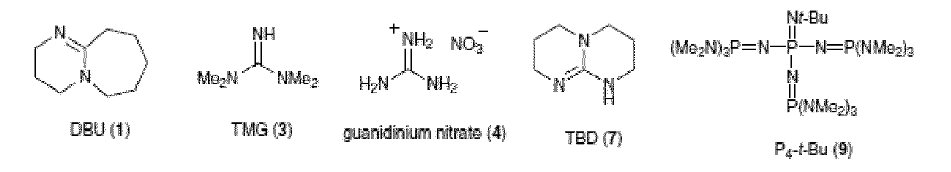

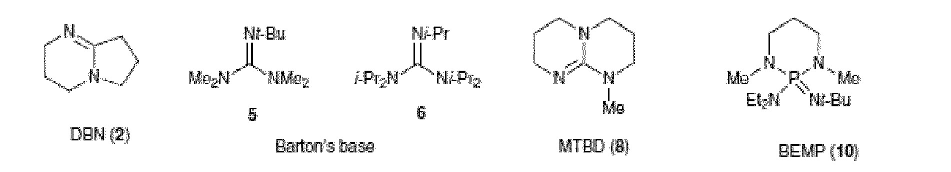

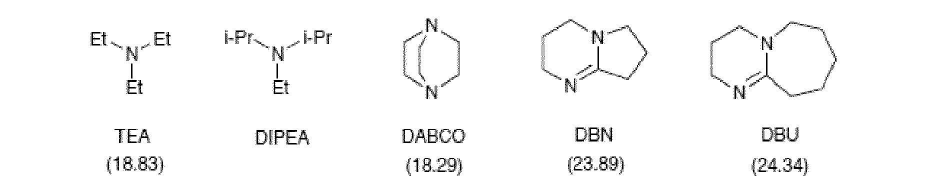

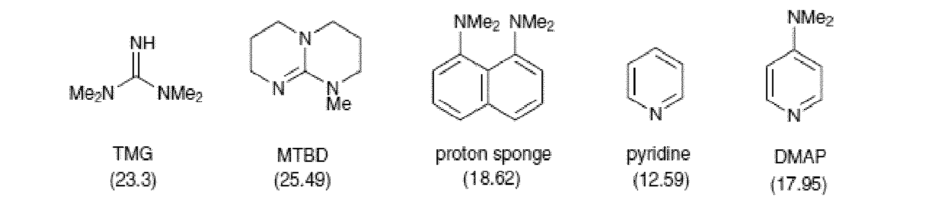

9. A process according to any of claim 1 to 5 wherein the catalyst is chosen from the group comprising 1, 8-Diazabicyclo [5,4,0] undec-7-ene (DBU), 1, 5-Diazabicyclo [4.3.0] non-5-ene (DBN), 1, 5, 7-Triazabicyclo [4.4.0] dec-5-ene (TBD), 7-methyl-1, 5, 7-Triazabicyclo [4.4.0] dec-5-ene (MTBD), 1, 1, 3, 3-tetramethylguanidine (TMG) or potassium bis(trimethylsilyl) amide (KHMDS). More preferably the catalyst is chosen between 1,5-diazabicyclo[4.3.0]non-5-ene (DBN) and 1,8-diazabicyclo[5.4.0]undec-7-ene (DBU).

10. A process according to one of claims 1 to 9 wherein the catalyst dosage is comprised between 0.1 to 10%, preferably between 0,5 to 5% of the total weight of the entire reaction system.

11. A process according to one of claims 1 to 10 wherein it comprises further adding a solvent chosen from the group comprising ethyl acetate, MIBK, N-methyl-pyrrolidone, acetone, tetrahydrofuran, dimethylformamide, dimethylacetamide, DMSO, sulfolane, toluene, xylene, benzene, cresol,.

12. A process according to one of claims 1 to 11 wherein the intermediate is decarboxylated at temperature from 20 to 150°C, preferentially from 30 to 120°C, and more preferentially from 40 to 100°C.

13. A process according to one of claims 1 to 12 wherein it comprises further adding stabilizers before, during or after production, said stabilizers being chosen from the group comprising N,N'-di-sec.-butyl-p-phenylendiamine (Kerobit BPD), 4,4'-Bis (α,α,dimethylbenzyl) diphenylamine, 4 isopropylamino diphenylamine, p-amino diphenylamine, N-(1,3-dimethylbutyl)-N'phenyl-p-phenylenediamine, N,N'-di-dec-butyl-p-phenylenediamine, mixtures of substituted p-diphenylamines, mixtures of octylated diphenylamines, ammonium N-nitrosophenylhydroxylamine, aluminium N-nitrosophenylhydroxylamine; salt of EDTA; HQ (hydroquinone), MEHQ (methylhydroquinone), BHT (butylated hydroxytoluene), BHA (butylated hydroxyanisole) ; 2,6-di-tert.-butyl-phenol (Kerobit TP 26), methoxyphenol, phenothiazine, 2, 6-di-tert-butyl-p-cresol or copper acetate, caustic soda or
hydroxyl tetramethyl piperidinyl oxy (HTPO).

14. A process for manufacturing N ethenyl N alkyl alkyl amide based polymer comprising polymerizing N alkyl alkyl amide monomers obtained according to the process of one of claims 1 and 13.

15. Use of polymer obtained according to claim 14 in water treatment, oil & gas recovery, paper, cosmetic, detergency, pharmaceutical, mining reagents, agriculture, drilling, construction.
